## Europäisches Patentamt

## European Patent Office

## Office européen des brevets

(11) Publication number: **0 130 672**
**B1**

(12) **EUROPEAN PATENT SPECIFICATION**

(45) Date of publication of patent specification: **22.02.89**

(51) Int. Cl.⁴: **C 07 C 11/04, C 07 C 7/11**

(21) Application number: **84302910.9**

(22) Date of filing: **01.05.84**

(54) Sorption fractionation process for olefin separation.

(30) Priority: **29.06.83 US 508779**

(43) Date of publication of application:
**09.01.85 Bulletin 85/02**

(45) Publication of the grant of the patent:
**22.02.89 Bulletin 89/08**

(84) Designated Contracting States:
**BE DE FR GB IT NL**

(56) References cited:
**US-A-2 745 889**

(73) Proprietor: **MOBIL OIL CORPORATION**
**150 East 42nd Street**
**New York New York 10017 (US)**

(72) Inventor: **Hsia, Chung Hweng**
**11 Somerset Place**
**Matawan, NJ 07747 (US)**
Inventor: **Owen, Hartley**
**5 Riverview Terrace**
**Belle Mead, NJ 08502 (US)**
Inventor: **Wright, Bernard Stanley**
**13 Shagbark Lane**
**East Windsor, NJ 08520 (US)**

(74) Representative: **Colmer, Stephen Gary**
**Patent Department c/o Mobil Services Company**
**Limited Mobil Court 3 Clements Inn**
**London WC2A 2EB (GB)**

**The file contains technical information submitted after the application was filed and not included in this specification**

Courier Press, Leamington Spa, England.

**Description**

This invention relates to a technique for separating light olefins to recover ethylene.

In the refining of petroleum and manufacture of fuels from fossil materials or various hydrocarbonaceous sources, an olefinic mixture is often produced. For instance, in cracking heavier petroleum fractions, such as gas oil, to make gasoline or distillate range products, light gases containing ethene, propene, butene and related aliphatic hydrocarbons are produced. It is known to recover these valuable by-products for use as chemical feedstocks for other processes, such as alkylation, polymerization, oligomerization and LPG fuel. Ethylene is particularly valuable as a basic material in the manufacture of polyethylene and other plastics, and its commercial value is substantially higher as a precursor for the chemical industry than as a fuel component. Accordingly, it is desirable to separate ethylene in high purity for such uses.

A typical by-product of fluid catalytic cracking (FCC) units is an olefinic stream rich in $C_2$—$C_4$ olefins, usually in mixture with lower alkanes. Ethylene can be recovered from such streams by conventional fractionation, such as cryogenic distillation, to recover the $C_2$ and $C_3$ + fractions; however, the equipment and processing costs are high. The present invention seeks to provide a process for economically separating ethylene from olefinic mixtures.

US—A—2,745,889 describes a procedure for processing light hydrocarbons produced by high temperature, low pressure cracking, including large proportions of $C_2$, $C_3$ and $C_4$ olefins. The process utilizes two simple absorbers in series which use lean oil absorbents. Although this process can effect total $C_4$ olefin separation and very good $C_3$ olefin separation, $C_2$ olefin yield is very low and can be improved only at the expense of $C_3$ olefin yield.

According to the invention, there is provided a fractionation process for recovering ethylene from a volatile olefinic feedstock comprising ethylene and $C_3$+ higher olefins by contacting the olefinic feedstock in a countercurrent sorption tower with a liquid sorbent stream comprising $C_6$+ gasoline boiling-range hydrocarbons to sorb selectively substantially all the $C_3$+ olefin components from the feedstock; withdrawing an ethylene-rich stream from the sorption tower; and contacting this ethylene-rich stream with a distillate boiling range liquid hydrocarbon stream in a sponge absorber to purify the ethylene-rich stream wherein the $C_3$+ olefin enriched $C_6$+ gasoline boiling range hydrocarbons are reboiled in reboiler loop *2J*; in that the ethylene-rich gas from upper gas outlet *2E* is mixed with the incoming liquid sorbent and this multi-phase mixture is passed into a phase separation *2F*, operatively connected between the primary sorption tower *2* and the secondary sponge absorber *3*; and in that the liquid sorbent from separator *2F* is pumped to the upper liquid inlet *2C* of the primary sorption tower *2*.

The sorption process may be operated under non-cryogenic conditions at moderate pressure and temperature using gasoline range hydrocarbons consisting essentially of $C_6$ to $C_{10}$ aliphatics.

The process of the invention is especially applicable to the separation of volatile hydrocarbons comprising a major amount of $C_2$—$C_4$ olefins, typically containing 10 to 50 mole% each of ethene and propene. In the detailed examples below the feedstock consists essentially of volatile aliphatic components as follows (on a mole% basis): ethene, 24.5%; propene, 46%; propane, 6.5%; 1-butene, 15%; and butanes, 8%, having an average molecular weight of about 42 and more than 85% olefins.

The gasoline sorbent is an aliphatic hydrocarbon mixture boiling in the normal gasoline range of about 50 to 165°C, with minor amounts of $C_4$—$C_5$ alkanes and alkenes. Preferably, the total gasoline sorbent stream to feedstock weight ratio is greater than about 3:1; however, the content of $C_3$+ olefinic components in the feedstock is a more preferred measure of sorbate to sorbent ratio. Accordingly, the process may be operated with a mole ratio of 0.2 moles to 10 moles of gasoline per mole of $C_3$+ hydrocarbons in the feedstock, with optimum operation utilizing a sorbent:sorbate molar ratio 1:1 to 1.5:1.

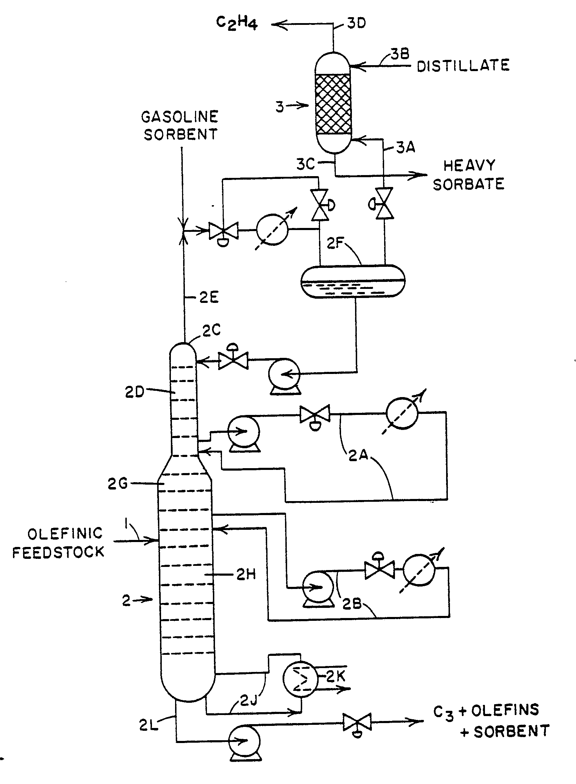

The process of the invention will now be described in greater detail by way of example only by reference to the accompanying drawing, which is a flow diagram of the process.

Referring to the drawing, olefinic feedstock is introduced to the system through a feedstock inlet 1 connected between stages of fractionating sorption tower 2 in which gaseous olefinic feedstock is contacted with liquid sorbent in a vertical fractionation column operating at least in the upper portion thereof is countercurrent flow. Effectively this unit is a $C_2/C_3$+ splitter. Design of sorption equipment and unit operations are established chemical engineering techniques, and generally described in Kirk-Othmer "Encyclopedia of Chemical Technology" 3rd Ed. Vol. 1 pp. 53—96 (1978). In conventional refinery terminology, the sorbent stream is sometimes known as lean oil.

Sorption tower 2, as depicted, has multiple contact zones, with the heat of absorbtion being removed via interstage pumps around cooling circuits 2A, 2B. The liquid gasoline sorbent is introduced to the sorption tower through an upper inlet 2C above the top contact section 2D. Incoming liquid sorbent is mixed with outgoing splitter overhead ethylene-rich gas from upper gas outlet 2E and to pass this multi-phase mixture into a phase separator 2F, operatively connected between the primary sorption tower 2 and a secondary sponge absorber 3. Liquid sorbent from separator 2F is then pumped to the upper liquid inlet 2C for countercurrent contact in a plate column or the like with upwardly flowing ethylene-rich vapors. Liquid from the bottom of upper contact zone 2D is pumped to a heat exchanger in loop 2A, cooled and returned to the tower above intermediate contact zone 2G, again cooled in loop 2B, and returned to the tower above contact zone 2H, which is located below the feestock inlet 1. Under tower design conditions of about 2100 kPa, it is preferred to maintain the liquid temperature of streams entering the tower from 2A, 2B and 2F at about 40°C. The lower contact zone 2H provides further fractionation of the olefin-rich liquid. Heat is supplied to the sorption tower by removing liquid from the bottom via reboiler loop 2J, heating this stream in heat exchanger 2K, and returning the reboiled bottom stream to the tower below contact zone 2H.

The liquid sorbate-sorbent mixture is withdrawn through bottom outlet 2L and pumped to storage or olefins recovery or to reaction. This stream is suitable for use as a feedstock in an olefins oligomerization unit or may be utilized as fuel products. Ethylene rich vapor from the primary sorption tower is withdrawn via separator 2F through conduit 3A.

Distillate lean oil is fed to the top inlet 3B of sponge absorber 3 under process pressure at ambient or moderately warm temperature (for example 40°C) and distributed at the top of a porous packed bed, such as Raschig rings, having sufficient bed height to provide multiple stages. The liquid rate is low; however, the sponge absorber permits sorption of about 25 weight percent of the distillate weight in $C_3+$ components sorbed from the ethylene-rich stream. This stream is recovered from bottom outlet 3C. It is understood that the sorbate may be recovered from the mixture with the sorbent by fractionation and the sorbent may be recycled or otherwise utilized. High purity ethylene is recovered from the system through gas outlet 3D and sent to storage, further processing or conversion to other products.

The sorption towers depicted in the drawing employ a plate column in the primary tower and a packed column in the secondary tower, however, the fractionation equipment may employ vapor-liquid contact means of various designs in each stage including packed beds of Raschig rings, saddles or other porous solids or low pressure drop valve trays (Glitsch grids). The number of theoretical stages will be determined by the feedstream composition, liquid:vapor (L/V) ratios, desired recovery and product purity. In the detailed example below, 17 theoretical stages are employed in the primary sorption tower and 8 stages in the sponge absorber, with olefinic feedstock being fed between the 7th and 9th stages of the primary sorption tower.

The following Examples illustrate the invention. They are based on the feedstock described above at 40°C and 2100 kPa supplied to stage 9 of the primary sorption tower. Gasoline is supplied at 85°C and 2150 kPa, and distillate lean oil is supplied at 40°C and 2100 kPa. Table I shows the conditions at each stage of the primary sorption tower, and Table II shows the conditions for the sponge absorber units for Example 1 (2 moles gasoline/mole of olefin in feedstock).

3

TABLE I

| Stage | Heat In (kW/tonne) | Temperature (°C) | Liquid/Vapor (L/V) Mole Ratio | Pressure (kPa) |
|---|---|---|---|---|
| 1 (top) | -121. + 362[1] | 37.8 | 6.947 | 2068.5 |
| 2 | | 38.5 | 2.245 | 2103.0 |
| 3 | | 39.7 | 2.222 | 2103.7 |
| 4 | | 42.3 | 2.227 | 2104.4 |
| 5 | | 47.2 | 2.221 | 2105.1 |
| 6 | | 54.2 | 2.185 | 2105.8 |
| 7 | - 29.[2] | 57.6 | 2.216 | 2106.5 |
| 8 | | 65.3 | 1.864 | 2107.2 |
| 9 | -820. + 120[3] | 59.9 | 2.447 | 2107.9 |
| 10 | | 67.7 | 1.954 | 2108.6 |
| 11 | | 71.8 | 1.814 | 2109.3 |
| 12 | | 74.1 | 1.743 | 2110.0 |
| 13 | | 75.4 | 1.704 | 2110.7 |
| 14 | | 77.0 | 1.684 | 2111.4 |
| 15 | | 80.5 | 1.644 | 2112.1 |
| 16 | | 92.3 | 1.541 | 2112.8 |
| 17 (bottom) | 400.[4] | 136.2 | 0.872 | 2116.3 |

(1)  Condenser Duty & Lean Oil

(2)  1st Heat Removal Duty

(3)  2nd Heat Removal Duty & Lean Oil

(4)  Reboiler Duty, based on tonnes of feedstock

TABLE II

| Stage | Heat In (kW/tonne) | Temperature (°C) | Liquid/Vapor (L/V) Mole Ratio | Pressure (kPa) |
|---|---|---|---|---|
| 1 | $2.9^{(1)}$ | 42.8 | 0.045 | 1999.6 |
| 2 | | 42.3 | 0.046 | 2000.2 |
| 3 | | 41.8 | 0.046 | 2000.9 |
| 4 | | 41.4 | 0.047 | 2001.6 |
| 5 | | 41.2 | 0.047 | 2002.3 |
| 6 | | 40.9 | 0.048 | 2003.0 |
| 7 | | 40.6 | 0.050 | 2003.7 |
| 8 | $32.8^{(2)}$ | 40.1 | 0.056 | 2004.4 |

(1)   Distillate Lean Oil

(2)   $C_2^=/C_3^{=+}$ Splitter Overhead

Examples 1 to 9

Based on the above design, the following data show the effects of varying the flow rate of gasoline absorbent in the primary tower $C_2/C_3^+$ splitter overhead and the corresponding effects of varying the distillate lean oil rate in the secondary sponge absorber. These data are shown in Table III, which give the ethylene $(C_2^=)$ recovery and purity from each of the primary and secondary sorption units.

## TABLE III

| Example No. | Gasoline Mole Ratio (1) | Distillate Mole Ratio | C2/C3$^+$ Splitter Overhead | | | Sponge Absorber Overhead | | |
|---|---|---|---|---|---|---|---|---|
| | | | C2$^=$ Recovery % | C2$^=$ Purity MOL% | WT% | C2$^=$ Recovery % | C2$^=$ Purity MOL% | WT% |
| 1 | 2:1 | 0.013 | 99.92 | 98.21 | 95.24 | 98.37 | 99.18 | 97.91 |
| 2 | 1:1 | 0.013 | 99.94 | 85.16 | 77.74 | 98.32 | 86.43 | 78.39 |
| 3 | 1.5:1 | 0.013 | 99.93 | 96.43 | 92.56 | 98.37 | 97.45 | 95.53 |
| 4 | 3:1 | 0.013 | 99.90 | 98.40 | 95.46 | 98.35 | 99.36 | 98.16 |
| 5 | 4:1 | 0.013 | 99.88 | 98.42 | 95.45 | 98.32 | 99.39 | 98.40 |
| 6 | 2:1 | 0.006 | 99.92 | 98.21 | 95.24 | 99.02 | 98.98 | 97.48 |
| 7 | 2:1 | 0.01 | 99.92 | 98.21 | 95.24 | 98.68 | 99.09 | 97.67 |
| 8 | 2:1 | 0.019 | 99.92 | 98.21 | 95.24 | 97.77 | 99.31 | 98.40 |
| 9 | 2:1 | 0.025 | 99.92 | 98.21 | 95.24 | 97.17 | 99.43 | 98.65 |

(1)  Gasoline Absorbent Rate Moles/Mole of Total Olefin in Feedstock.

In general, as the flow rate of lean oil increases, the ethylene recovery decreases, while the purity increases. The data for the splitter/absorber combination show that the excellent results are obtained with a gasoline mole ratio of at least 1:1 (based on $C_3^+$ hydrocarbons). Such conditions will result in a $C_2^=$ recovery of greater than 98%. Purity of more than 99 mole% can be achieved with a gasoline ratio of at least 2:1.

A preferred sorbent source is olefinic gasoline and distillate produced by catalytic oligomerization according to U.S. Patent 4,211,640 and U.S. Patent Application Serial No. 488,834. The $C_3^+$ olefin sorbate and gasoline may be fed directly to such an oligomerization process, with a portion of the recovered gasoline and distillate being recycled to the sorption fractionation system. Table IV shows the boiling range fraction composition for typical gasoline and distillate sorbents.

### Table IV

#### Lean Oil Compositions (MOL %)

| | Gasoline | Distillate |
|---|---|---|
| Propane | 0.00 | 0 |
| Isobutane | 0.15 | 0 |
| 1-Butene | 0.12 | 0 |
| N-Butene | 0.59 | 0 |
| Isopentane | 2.60 | 0 |
| 1-Pentene | 0.24 | 0 |
| N-Pentane | 0.24 | 0 |
| 52- 82°C | 11.24 | 0 |
| 82-104°C | 22.02 | 0 |
| 104-127°C | 23.54 | 0.02 |
| 127-138°C | 11.23 | 0.09 |
| 138-149°C | 10.47 | 0.43 |
| 149-160°C | 8.70 | 2.00 |
| 160-171°C | 1.54 | 2.13 |
| 171-182°C | 0.92 | 7.06 |
| 182-193°C | 0.31 | 11.16 |
| 193-204°C | 0.10 | 14.53 |
| 204-216°C | 0.01 | 8.36 |
| 216-227°C | 0.00 | 8.56 |
| 227-238°C | 0 | 7.56 |
| 238-249°C | 0 | 6.50 |
| 249-260°C | 0 | 6.00 |
| 260-271°C | 0 | 4.30 |
| 271-293°C | 0 | 5.10 |
| 293-316°C | 0 | 4.13 |
| 316-338°C | 0 | 3.24 |
| 338-360°C | 0 | 3.17 |
| 360-382°C | 0 | 4.63 |
| 382-404°C | 0 | 0.91 |
| 404-438°C | 0 | 0.11 |

The sponge absorber may be constructed in a separate unit, as shown, or this operation may be conducted in an integral shell vessel with the main fractionation unit. In the alternative integral design, the rich sponge oil may be recovered from the upper contact zone as a separate steam, or the heavy distillate sorbent may be intermixed downwardly with gasoline sorbent and withdrawn from the bottom of the main fractionation zone.

## Claims

1. A fractionation process for recovering ethylene from a volatile olefinic feedstock comprising ethylene and $C_3+$ higher olefins by contacting the olefinic feedstock in a countercurrent sorption tower with a liquid sorbent stream comprising $C_6+$ gasoline boiling-range hydrocarbons to sorb selectively substantially all the $C_3+$ olefin components from the feedstock; withdrawing an ethylene-rich stream from the sorption tower; and contacting this ethylene-rich stream with a distillate boiling range liquid hydrocarbon stream in a sponge absorber to purify the ethylene-rich stream characterised in that the $C_3+$ olefin enriched $C_6+$ gasoline boiling range hydrocarbons are reboiled in reboiler loop *2J*; in that the ethylene-rich gas from upper gas outlet *2E* is mixed with the incoming liquid sorbent and this multi-phase mixture is passed into a phase separation *2F*, operatively connected between the primary sorption tower *2* and the secondary sponge absorber *3*; and in that the liquid sorbent from separator *2F* is pumped to the upper liquid inlet *2C* of the primary sorption tower *2*.

2. A process according to claim 1, wherein olefinic liquid gasoline is supplied to the sorption tower in a molar ratio of 0.2:1 to 10:1 based on feedstock $C_3+$ olefins;
the olefinic feedstock comprises from 10 to 50 mole% ethylene and from 10 to 50 mole% propene; and
purified ethylene product is recovered having an average molecular weight not greater than 28.5.

3. A process according to claim 1 or claim 2, wherein the distillate liquid stream is supplied to the sponge absorber in a molar ratio of 0.01:1 to 0.06:1 based on ethylene whereby $C_3+$ and gasoline range hydrocarbons escaping the first sorption tower with the ethylene-rich stream are absorbed by the distillate liquid.

4. A process according to any one of claims 1 to 3, wherein fractionation is carried out under non-cryogenic conditions.

5. A process according to any one of claims 1 to 4, wherein the feedstock comprises a major amount of $C_2$—$C_4$ olefins.

6. A process according to any one of claims 1 to 5, wherein the gasoline sorbent is fed to the sorption tower in a weight ratio based on total olefinic feedstock of at least 3:1.

7. A process according to any one of claims 1 to 6, operated under a pressure of 2000 to 2200 kPa.

## Patentansprüche

1. Fraktionierungsverfahren zur Gewinnung von Ethylen aus einem flüchtigen olefinischen Ausgangsmaterial, welches Ethylen und höhere $C_3+$-Olefine umfaßt, durch Kontakt dieses olefinischen Ausgangsmaterials in einer Gegenstromsorptionskolonne mit einem flüssigen Sorptionsmittelstrom, der $C_6+$-Kohlenwasserstoffe im Benzinsiedebereich umfaßt, um im wesentlichen die gesamten $C_3+$-Olefinkomponenten aus diesem Augangsmaterial selektiv zu sorbieren; Abziehen eines ethylenreichen Stromes aus der Sorptionskolonne und Kontakt dieses ethylenreichen Stromes mit einem flüssigen Kohlenwasserstoffstrom, im Destillatsiedebereich in einem Schwammabsorber, um den ethylenreichen Strom zu reinigen, dadurch gekennzeichnet, daß die mit $C_3+$-Olefin angereicherten $C_6+$-Kohlenwasserstoffe im Benzinsiedebereich in einer Reboilerschlange (2J) aufgekocht werden, daß das ethylenreiche Gas vom oberen Gasauslaß (2E) mit dem ankommenden flüssigen Sorptionsmittel vermischt wird, und diese Mehrphasenmischung in die Phasentrennung (2F) geleichtet wird, die wirksam zwischen der primären Sorptionskolonne (2) und den sekundären Schwammabsorber (3) eingebunden ist, und daß das flüssige Sorptionsmittel vom Separator (2F) zum oberen Flüssigkeitseinlaß (2C) der primären Sorptionskolonne (2) gepumpt wird.

2. Verfahren nach Anspruch 1, worin das olefinische flüssige Benzin bezogen auf die $C_3+$-Olefine des Ausgangsmaterials der Sorptionskolonne in einem Molverhältnis von 0,2:1 bis 10:1 zugeführt wird das olefinische Ausgangsmaterial von 10 bis 50 Mol.-% Ethylen und von 10 bis 50 Mol.-% Propylen umfaßt und das gereinigte Ethylenprodukt mit einem durchschnittlichen Molekulargewicht von nicht größer als 28,5 gewonnen wird.

3. Verfahren nach Anspruch 1 oder Anspruch 2, worin der flüssige Destillatstrom bezogen auf Ethylen den Schwammabsorber in einem Molverhältnis von 0,01:10 bis 0,06:1 zugeführt wird, wodurch $C_3+$ und Kohlenwasserstoff im Benzinbereich, die die erste Sorptionskolonne mit dem ethylenreichen Strom verlassen, durch die Destillatflüssigkeit absorbiert werden.

4. Verfahren nach einem der Ansprüche 1 bis 3, worin die Fraktionierung unter nicht cryogenen Bedingungen durchgeführt wird.

5. Verfahren nach einem der Ansprüche 1 bis 4, worin das Ausgangsmaterial eine Hauptmenge an $C_2$ bis $C_4$-Olefinen umfaßt.

8

# EP 0 130 672 B1

6. Verfahren nach einem der Ansprüche 1 bis 5, worin das Benzinsorptionsmittel bezogen auf das gesamte olefinische Ausgangsmaterial der Sorptionskolonne in einem Gewichtsverhältnis von mindestens 3:1 zugeführt wird.

7. Verfahren nach einem der Ansprüche 1 bis 6, das bei einem Druck von 200 bis 2200 kPa durchgeführt wird.

**Revendications**

1. Un procédé de fractionnement pour la récupération d'éthylène à partir d'une charge oléfinique volatile, comprenant de l'éthylène et des oléfines supérieures en $C_3+$, par mise au contact de la charge oléfinique, dans une tour de sorption à contre-courant d'un courant de sorbant liquide comprenant des hydrocarbures du domaine d'ébullition des essences en $C_6+$ pour absorber sélectivement, presque tous les constituants oléfiniques en $C_3+$ de la charge, par extraction d'un courant enrichi en éthylène de la tour de sorption; et par contact de ce courant enrichi en éthylène avec un courant d'hydrocarbures liquides du domaine d'ébullition des distillats dans un absorbeur spongieux pour purifier le courant enrichi en éthylène, caractérisé en ce que les hydrocarbures du domaine d'ébullition de l'essence en $C_6+$ enrichis en oléfines $C_3+$ sont remis en ébullition dans une boucle de rebouilleur (2J); en ce que le courant enrichi en éthylène provenant de la sortie supérieure de gaz (2E) est mélangé avec le sorbant liquide entrant et en ce que ce mélange multiphase traverse une zone de séparation de phase (2F), activement connectée entre la tour de sorption primaire (2) et l'absorbeur spongieux secondaire (3), et en ce que le sorbant liquide provenant du séparateur (2F) est pompé vers l'entrée de liquide supérieure (2C) de la tour de sorption primaire (2).

2. Un procédé selon la revendication 1, dans lequel l'essence liquide oléfinique est alimenté à la tour de sorption selon un rapport molaire de 0,2/1 à 10/1, rapporté aux oléfines $C_3+$ de la charge; la charge oléfinique comprend de 10 à 50 moles% d'éthylène et de 10 à 50 moles% de propène; et l'éthylène produite purifiée est récupérée avec un poids moléculaire moyen non supéieur à 28,5.

3. Un procédé selon la revendication 1 ou la revendication 2, dans lequel le courant de liquide de distillat est introduit dans l'absorbeur spongieux selon un rapport molaire de 0,01/1 à 0,06/1, rapporté à l'éthylène, de sorte que les hydrocarbures du domaine de l'essence en $C_3+$ s'échappant de la première tour de sorption avec le courant enrichi en éthylène soient absorbés par le liquide du distillat.

4. Un procédé selon l'une des revendications 1 à 3, dans lequel le fractionnement est effectué sous des conditions non cryogéniques.

5. Un procédé selon l'une quelconque des revendications 1 à 4, dans lequel la charge comprend une proportion majeure d'oléfines en $C_2$—$C_4$.

6. Un procédé selon l'une quelconque des revendications 1 à 5, dans lequel le sorbant à base d'essence est amené à la tour de sorption selon un rapport en poids, rapporté à la charge oléfinique totale, d'au moins 3/1.

7. Un procédé selon l'une quelconque des revendications 1 à 6, mis en oeuvre sous une pression de 2 000 à 2 200 kPa.

9